# EUROPEAN PATENT SPECIFICATION

(11) **EP 1 208 387 B1**
(45) Date of publication and mention of the grant of the patent: **31.03.2004**
(21) Application number: 00956662.1
(22) Date of filing: 30.08.2000
(51) Int. Cl.: G01R 31/08

(54) **APPARATUS AND METHOD OF DETECTING AND CONTROLLING TWISTS IN MULTICORE CABLES**
VORRICHTUNG UND VERFAHREN ZUR ERMITTLUNG UND STEUERUNG VON VERSEILUNGEN IN MEHRADRIGEN KABELN
APPAREIL ET PROCEDE PERMETTANT DE DETECTER ET DE VERIFIER LES TORSIONS DE CABLES MULTICONDUCTEURS

(30) Priority: 01.09.1999 GB 9920588
(43) Date of publication of application: 29.05.2002
(73) Proprietor: Beta LaserMike Limited, High Wycombe, Buckinghamshire HP12 3RT (GB)
(72) Inventor: FLEMING, Patrick, Oxon RG9 1ND (GB); HASSAN, Halil, Giray, Edgware HA8 6RX (GB)
(74) Representative: Enskat, Michael Antony Frank
(86) International application number: PCT/GB2000/003302
(87) International publication number: WO 2001/016608

(56) References cited:
- EP-A- 0 608 993
- US-A- 4 584 875
- US-A- 4 648 054
- PATENT ABSTRACTS OF JAPAN vol. 013, no. 149 (P-855), 12 April 1989 (1989-04-12) -& JP 63 311110 A (SUMITOMO ELECTRIC IND LTD), 19 December 1988 (1988-12-19)

## Description

The present invention relates to methods and apparatus for detecting and controlling twists in multicore cables.

Cables used for telecommunication and other high technology applications are required to be manufactured to high specifications since the way in which two or more conductors are twisted together can effect attenuation and crosstalk.

Generally, cables which have two or more conductors twisted together rely on the apparatus generating the twist to ensure that the twisting takes place in a regular and uniform manner. However, in practice, the twist produced will vary and this in turn will vary the attenuation within the conductors and the crosstalk between them.

US Patent No 4,648,054 discloses a yam twist measurement device having a freely rotatable disc with its axis parallel to the axis of the travelling yam. By measuring the circumferential displacement of the disc and the corresponding linear translation of the yam, the amount of twist on the surface of the yam can be calculated.

It is an object of the present invention to provide apparatus and method of measuring the twist in a twisted cable as it is being manufactured so that with the aid of feedback, the twisting action can be modified to reduce non-uniformity towards zero.

According to the present invention, there is provided an apparatus according to claim 1.

As will further be described, a method according to claim 2 is also provided.

Apparatus and methods for detecting and controlling the twists in multicore cables, will now be described, by way of example, with reference to the accompanying diagrammatic drawings, in which:
Figure 1 is a plan view of the apparatus;
Figure 2 is a front elevation of the apparatus of Figure 1;
Figure 3 is a view from one side of the optical detection system of the apparatus of Figures 1 and 2; and
Figure 4 is a front elevation of an apparatus embodying the invention.

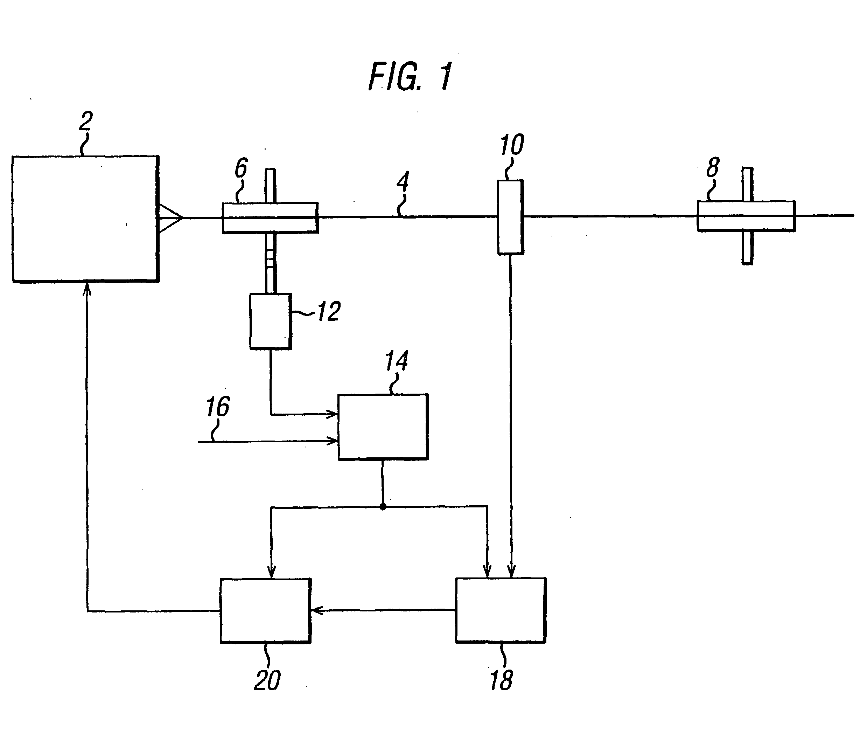

Figure 1 shows part of the twisted cable production line. Individual strands or conductors are taken from separate supply reels (not shown) and fed through a twisting assembly 2 in which orbital rotary components (not shown) produce a twist in the cable. The twisted cable 4 emerging from the assembly 2 passes over a pair of spaced supporting wheels 6 and 8. A detection arrangement 10 straddles the cable as it passes between the wheels 6 and 8.

The shaft of the wheel 6 is coupled to a transducer 12 which provides an output proportional to the speed of the wheel which in turn is dependent upon the speed of travel of the cable 4.

The output of the transducer 12 is fed to a calibration unit 14. The calibration unit has an adjustable input 16 which can be set to the nominal number of 360° twists that the twisting assembly induces per unit length of the cable. The frequency f_{ref} of the output signal of the calibration unit is thus arranged to equal nominal rate or frequency at which the conductors turn about each other (the twist frequency) as they pass over the wheel 6.

A detection assembly 10 downstream of the wheel 6 measures the variation in the lateral dimension of the cable as the conductors twist about each other and the resultant signal produced will include a number of frequency components including the actual twist frequency of the cable. The output of the detection assembly 10 is fed to an analyser 18 which conducts a Fourier analysis on the input signal. The analyser 18 also receives the reference frequency f_{ref} which it uses to establish a bandwidth to select only the actual twist frequency component fₜ from the multitude of different frequency components established by the Fourier analysis.

Other types of analysis may be used, eg timing analysis.

This twist frequency component fₜ is fed together with the reference frequency f_{ref} to comparator 20 which produces a difference signal f_{d}. The difference signal of frequency f_{d} is fed back to the twisting assembly which responds by adjusting the twisting action in a sense to reduce the difference to zero.

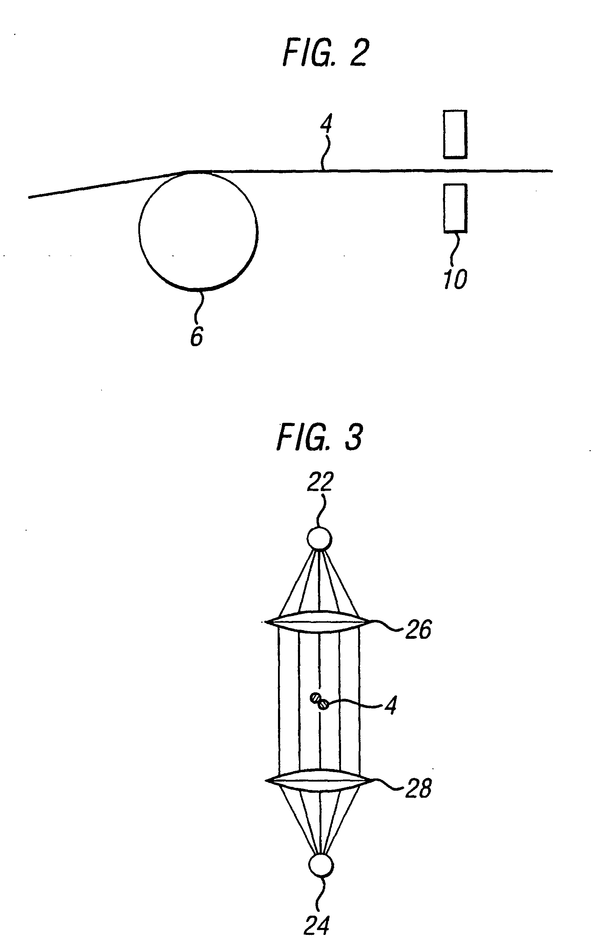

Figure 3 shows the detection assembly 10 in more detail. As shown, a light emitter 22, on one side of the cable 4, is directed at a light receiver 24 in the opposite side of the cable. A first lens 26 located between the emitter 22 and the cable produces parallel rays of light, some of which are interrupted by the cable 4. Another lens 28 between the cable 4 and the receiver 24 receives the non-intercepted light and focuses the rays on the receiver 24.

As can be seen as the twist progresses, the amount of light intercepted by the cable will vary and so will the shadow cast by the light on the receiver 24. Hence, the output signal from the receiver will have a frequency component equal to the twist frequency.

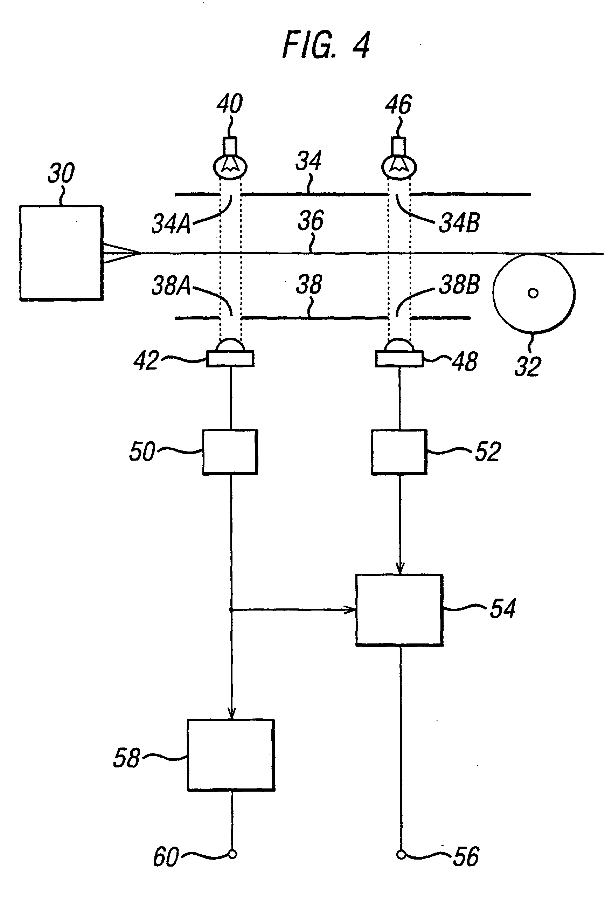

The apparatus shown in Figure 4 is arranged to provide a first output indicative of the twist rate of a cable consisting of twin twisted strands or conductors and a second output indicative of the speed of the cable. Both of these parameters can be used in feedback systems to control the production of the cable.

As shown, the twisted cable 36, emerging from a twisting assembly 30, is supported by a downstream roller 32. A light shield 34 extending above the cable 36 is provided with two slots 34A and 34B spaced apart in the longitudinal direction of the cable 36 and extending tangential to the cable.

A light shield 38 extending below the cable 36 is also provided with two slots 38A and 38B spaced apart in the longitudinal direction of the cable and extending tangential to the cable. The slots 34A and 34B are in direct alignment with respective slots 38A and 38B.

A light source 40 projects a beam of light through slots 34A and 38A and a photo-detector 42 receives the light emerging from the slot 38A. Similarly, a light source 46 projects a beam of light through the slots 34B and 38B and a photo-detector 48 receives the light emerging from the slot 38B.

A filter 50 is connected to receive the output from the detector 42 and passes a signal having a frequency over a specific range.

A filter 52, similar to the filter 50, is connected to receive the output of the photo-detector 48. A phase comparator 54 is connected to the outputs of the two filters 50 and 52 and provides a phase difference or error signal at an output terminal 56.

A processor 58 receives the output of the filter 50 to provide a speed or speed error signal at output terminal 60.

In operation, as the twisted cable passes between respective pairs of slots 34A, 38A and 34B and 38B, it will present a varying profile and so the shadow it casts on respective photo-detectors 42 and 48, will vary in a generally sinusoidal manner. The output signal from the detectors will thus include a selected frequency component related to the speed of the cable, assuming the twist rate remains constant. Any variation in the twist rate will manifest itself in a phase change in selected frequency components in the outputs of the two detectors 42 and 48.

The two filters 50 and 52 are arranged to have a relatively narrow passband having a centre frequency corresponding to the nominal twist frequency of the cable when run at nominal speed. The processor 58, upon receiving the output signal from the filter 50, coverts it into a speed signal which is then fed to the output 60. Instead, the processor 58 may compare the output signal from the filter 50 with a nominal value and then feed an error signal to the output 60.

The phase comparator 54 compares the phases of the two output signals from the filters 50 and 52 and provides a difference signal at output 56. Instead, the comparator may compare the phase difference with a nominal phase difference and feed an error signal to the terminal 56.

The signals at the outputs 60 and 56 can be fed back to the assembly 30 to maintain the speed and twist rate of the cable substantially constant.

It will be appreciated that while the detection assembly is described as an optical sensor, other sensors which can detect a change in the twist of the cable can equally be used, for example, a capacitive or ultrasonic detection system.

In some embodiments, it may not be necessary to determine the nominal twist frequency and twist rate for a cable.

## Claims

1. Apparatus for detecting speed and twist rate variations in a cable (36) having at least two twisted elongate elements and travelling along a predetermined path, the apparatus comprising a first sensor having a light source (40) and a first detector means (42) positioned about said path so that the cable (36) interrupts the light path from the source (40) to the first detector means (42) to cast a varying shadow on the first detector means (42) as the cable (36) travels along the predetermined path, a second sensor spaced apart along said path from said first sensor, the second sensor having a light source (46) and a second detector means (48) positioned about said path so that the cable (36) interrupts the light path from the source (46) to the second detector means (48) to cast a varying shadow on the second detector means (48) as the cable (36) travels along the predetermined path, and means (58, 54) for respectively processing the outputs of the two detector means (42, 48) which are spaced apart a predetermined distance and which provide at a first output (60) a signal indicative of the speed of the cable and at a second output (56) a signal representing a phase difference between said outputs of said two detector means and indicative of variations in the twist rate of the cable.

2. A method of detecting the speed and twist rate variations in a cable having at least two twisted elongate elements and travelling along a predetermined path, the method comprising the step of monitoring the variation in profile of the cable (36) as it passes a first location along said path to provide a first measurement signal, the step of monitoring the variation in profile of the cable (36) as it passes a second location along said path to provide a second measurement signal, spacing the second location a predetermined distance from said first location so that the first and second measurement signals can then be processed in a further step to produce therefrom said signal indicative of the actual speed of the cable and a signal representing a phase difference between said first and second measurement signals and indicative of variations in the actual twist rate of the cable.

## Patentansprüche

1. Vorrichtung zum Feststellen von Geschwindigkeits- und Drallgeschwindigkeitsvariationen in einem Kabel (36) mit wenigstens zwei verdrillten Langelementen und Bewegung entlang einem vorbestimmten Weg, wobei die Vorrichtung einen ersten Sensor mit einer Lichtquelle (40) und ein erstes Detektormittel (42) umfasst, die um besagten Weg herum positioniert sind, so dass das Kabel (36) den Lichtweg ab der Quelle (40) zum ersten Detektormittel (42) unterbricht, um einen variierenden Schatten auf das erste Detektormittel (42) zu werfen sowie sich das Kabel (36) entlang dem vorbestimmten Weg bewegt, ein zweiter Sensor entlang besagtem Weg mit Abstand vom besagten ersten Sensor angeordnet ist, der zweite Sensor eine Lichtquelle (46) aufweist und ein zweites Detektormittel (48) so um besagten Weg herum positioniert ist, dass das Kabel (36) den Lichtweg ab der Quelle (46) zum zweiten Detektormittel (48) unterbricht, um einen variierenden Schatten auf das zweite Detektormittel (48) zu werfen sowie sich das Kabel (36) entlang dem vorbestimmten Weg bewegt, und Mittel (58, 54) um jeweils die Ausgaben der zwei Detektormittel (42, 48) zu verarbeiten, die eine vorbestimmte Entfernung von einander angeordnet sind, und die an einem ersten Ausgang (60) ein Signal, das auf die Geschwindigkeit des Kabels schließen lässt und an einem zweiten Ausgang (56) ein Signal bereitstellen, das eine Phasendifferenz zwischen besagten Ausgaben besagter zwei Detektormittel repräsentiert und auf Variationen in der Drallgeschwindigkeit des Kabels schließen lässt.

2. Ein Verfahren zum Feststellen der Geschwindigkeits- und Drallgeschwindigkeitsvariationen eines Kabels mit wenigstens zwei verdrillten Langelementen, die sich entlang einem vorbestimmten Weg bewegen, wobei das Verfahren den Schritt der Überwachung der Profilvariation des Kabels (36) sowie es eine erste Position entlang dem besagten Weg passiert, um ein erstes Messsignal bereitzustellen, den Schritt der Überwachung der Profilvariation des Kabels (36) sowie es eine zweite Position entlang dem besagten Weg passiert, um ein zweites Messsignal bereitzustellen, Anordnen der zweiten Position in einer vorbestimmten Entfernung von besagter ersten Position umfasst, so dass die ersten und zweiten Messsignale dann in einem weiteren Schritt verarbeitet werden können, um daraus besagtes Signal, das auf die tatsächliche Geschwindigkeit des Kabels schließen lässt und ein Signal, das eine Phasendifferenz zwischen besagten ersten und zweiten Messsignalen repräsentiert, und das auf Variationen in der eigentlichen Drallgeschwindigkeit des Kabels schließen lässt, zu produzieren.

## Revendications

1. Appareil permettant de détecter les variations de vitesse et le taux de torsion d'un câble (36) ayant au moins deux éléments allongés torsadés et passant sur un trajet prédéterminé, l'appareil comprenant un premier capteur ayant une source de lumière (40) et un premier moyen de détection (42) disposé sur ce trajet de façon à ce que le câble (36) interrompe le trajet de la lumière allant de la source (40) vers le premier moyen de détection (42) en jetant une ombre variable sur le premier moyen de détection (42) quand le câble chemine le long du trajet prédéterminé; un deuxième capteur, disposé sur ce même trajet à une certaine distance du premier détecteur, le deuxième capteur ayant une source de lumière (46) et un deuxième moyen de détection (48) disposé sur ce trajet de façon à ce que le câble (36) interrompe le trajet de la lumière allant de la source (46) vers le deuxième moyen de détection (48) en jetant une ombre variable sur le deuxième moyen de détection (48) quand le câble (36) chemine le long du trajet prédéterminé, et un moyen (58, 54) pour traiter respectivement les sorties des deux moyens de détection (42, 48) disposés à une distance prédéterminée l'un de l'autre et qui donnent, à une première sortie (60), un signal indiquant la vitesse du câble et à une deuxième sortie (56) un signal représentant une différence de phase entre les sorties des deux moyens de détection et indiquant les variations dans le taux de torsion du câble.

2. Une méthode pour détecter la vitesse et le taux de variation de torsion d'un câble ayant au moins deux éléments allongés torsadés et cheminant le long d'un trajet prédéterminé, la méthode comprenant l'étape de surveiller la variation de profil du câble (36) quand il passe un premier poste sur ce trajet pour donner un premier signal de mesure, l'étape de surveillance de la variation de profil du câble (36) quand il passe un deuxième poste le long dudit trajet pour donner un deuxième signal de mesure, le deuxième poste étant disposé à une distance prédéterminée du premier poste de sorte que le premier et le deuxième signal de mesure peuvent alors être traités en une troisième étape pour produire le signal indiquant la vitesse réelle du câble et un signal représentant une différence de phase entre le premier et le deuxième signal de mesure et indiquant les variations de taux réel de torsion du câble.
